# EUROPEAN PATENT APPLICATION

(11) **EP 4 818 701 A1**
(43) Date of publication of application: **07.10.2026**
(21) Application number: 25382335.5
(22) Date of filing: 03.04.2025
(51) Int. Cl.: G01N 33/58, G01N 33/68, G01N 33/96

(54) **RECOMBINANT PROTEIN AND KIT FOR USE IN THE QUANTIFICATION OF ANTIBODIES IN IMMUNOASSAYS**

(71) Applicant: Fundació Privada Institut de Recerca Sobre Immunopatologies-Caixa Irsicaixa, 08916 Badalona, Barcelona (ES)
(72) Inventor: CARRILLO MOLINA, Jorge, E-08916 Badalona (Barcelona) (ES); PEDREÑO LÓPEZ, Nuria, E-08916 Badalona (Barcelona) (ES); RODRÍGUEZ DE LA CONCEPCIÓN, María Luisa, E-08916 Badalona (Barcelona) (ES); MATILLA MARTÍNEZ, Marina, E-08916 Badalona (Barcelona) (ES)
(74) Representative: ABG Intellectual Property Law, S.L.

(57) **Abstract**

The present invention relates to a recombinant protein comprising the variable part of an antibody against a specific tag and the Fc region of an antibody for use as quantitation standard in an immunoassay for antibodies against antigens tagged with that specific tag.

## Description

### FIELD OF THE INVENTION

The present invention belongs to the field of Biotechnology and relates to a universal standard for antigen-specific antibody quantification. The present invention relates to a recombinant protein comprising the variable part of an antibody against a specific tag and the Fc region of an antibody for use as quantitation standard in an immunoassay for antibodies against antigens tagged with that specific tag.

### BACKGROUND

Immunoassays, such as the Enzyme-Linked ImmunoSorbent Assay (ELISA), are routinely and widely used in the laboratory for the determination of the levels of several analytes in biological fluids (i.e. plasma, blood, urine, saliva), including antigen-specific antibody titers. The quantification of these antibodies is crucial for monitoring the humoral response developed in the time course of a disease or after vaccination.

Most immunoassays are either qualitative or semiquantitative, since they inform about sample positivity status, or provide arbitrary results based on their own reference standard, respectively. To get quantitative data that can be used to directly compare the antibody levels elicited against different antigens is challenging, mainly due to the lack of an adequate standard that allows the comparison of heterologous responses.

Tang, et al. address this problem in npj Vaccines volume 9, Article number: 8 (2024), and developed a generalizable method known as *MASCALE* (Mass Spectrometry Enabled Conversion to Absolute Levels of *E*LISA Antibodies) for absolute quantitation of antibodies by calibrating ELISA reference sera using mass spectrometry (Tang, C., Verwilligen, A., Sadoff, J. et al. Absolute quantitation of binding antibodies from clinical samples. npj Vaccines 9, 8 (2024). https://doi.org/10.1038/s41541-023-00793-w).
The present invention provides a simpler and easier to use solution to this problem.

### SUMMARY OF THE INVENTION

A first aspect of the present invention relates to a recombinant protein comprising the variable part of an antibody against a specific tag and the Fc region of an antibody, for use as quantitation standard in an immunoassay for antibodies against antigens tagged with that specific tag.

A second aspect of the present invention relates to a nucleic acid encoding for the recombinant protein of the first aspect, for use as quantitation standard in an immunoassay for antibodies against antigens tagged with that specific tag.

A third aspect of the present invention relates to a cell comprising the nucleic acid of the second aspect, for use as quantitation standard in an immunoassay for antibodies against antigens tagged with that specific tag.

A fourth aspect of the present invention relates to a kit of parts comprising at least two recombinant proteins of the first aspect, or at least two nucleic acids of the second aspect, or at least two cells of the third aspect, wherein each recombinant protein comprises the variable part of an antibody against the same specific tag and the Fc region of each recombinant protein is from an antibody with a different isotype.

A fifth aspect of the present invention relates to the kit of the fourth aspect for use as quantitation standard in an immunoassay for antibodies against antigens tagged with that specific tag.

### DETAILED DESCRIPTION OF THE INVENTION

The inventors of the present invention have developed a standard system based on recombinant proteins that combine a variable region of an antibody that recognizes a specific tag (such as His-tag) with the Fc region of an antibody from the same or different species, for use as quantitation standard in an immunoassay for antibodies against antigens tagged with that specific tag. For example, the recombinant proteins have been developed with Fc regions of all human immunoglobulin (Ig) isotypes (IgM, IgD, IgA, IgG, and IgE), including all IgG and IgA subclasses (IgG1, IgG2, IgG3, IgG4, IgA1, and IgA2). Therefore, since the recombinant proteins have the same specificity, they can be used as standards allowing the direct comparison of the results obtained with different immunoassays that use the same tagged (e.g. His-tag) antigens from different origins.

In addition, these standards allow the characterization of the isotypic composition of the humoral response elicited against any antigen.

The epitopes recognized by the recombinant proteins of the invention can be used in a wide array of immunoassays, including those that use native (i.e. ELISA) or denatured antigens (i.e. Western blot).

Thus, the present standard system has the advantage of allowing the quantification of the immune response in a variety of immunoassays, in a simple and universal manner, since it can be used for any antigen which can be tagged. Importantly, tagged antigens are usually commercially available, as it is common to use tags in the purification process and these tags are useful for research.

As used herein, the term "tag" or "tagged" means any biochemical moiety that has a well-characterized, readily available detection methodology, such as an amino acid sequence that is specifically recognized by an antibody and can be a peptide or a protein. The skilled person can attach said tag(s) to other molecules to flag them. They can be added to either end of the target protein, so they are either C-terminus or N-terminus specific or are both C-terminus and N-terminus specific. Some tags are also inserted at sites within the protein of interest; they are known as internal tags. Tags are widely used in biological assays to assist in protein detection and purification. Typically, in such detection, quantification and purification methods, a protein must be cloned and then genetically fused with one or more tags. The presence of the tag or tags allows for highly specific and sensitive detection of the protein to which it is fused, using an anti-tag antibody. This investigative approach has enabled the detection of the epitope-protein fusions in various biological samples, including in cells, tissues, biological fluids, animals, and lysates. Epitope tags are also used in protein purification, in so-called pulldown assays.

Many tags have been described. For the present invention, any tag which can be recognized by specific antibodies against said tag can be used. Examples of peptide tags are ALFA-tag, an helical peptide tag (SEQ ID NO: 51); AviTag, a peptide (SEQ ID NO: 52) allowing biotinylation by the enzyme BirA so the protein can be isolated by streptavidin; C-tag, a peptide that binds to a single-domain camelid antibody developed through phage display; Calmodulin-tag or Calmodulin binding protein tag or CBP-tag, a peptide (SEQ ID NO: 53) bound by the protein calmodulin; polyglutamate tag, a peptide consisting of six glutamate residues, binding efficiently to anion-exchange resin such as Mono-Q; polyarginine tag, a peptide binding efficiently to cation-exchange resin (from 5 to 9 consecutive arginine residues); E-tag, (SEQ ID NO: 54); FLAG-tag, (SEQ ID NO: 55); HA-tag, a peptide from hemagglutinin recognized by an antibody (SEQ ID NO: 56); His-tag, a peptide of between 5 and10 histidine residues; Gly-His-tags, which are N-terminal His-Tag variants including one G residue before the His-tag; Myc-tag, (SEQ ID NO: 57), a peptide derived from c-myc gene; NE-tag, an 18-amino-acid synthetic peptide (SEQ ID NO: 58); Rho1D4-tag, the last 9 amino acids of the intracellular C-terminus of bovine rhodopsin (SEQ ID NO: 59); S-tag, a peptide derived from Ribonuclease A (SEQ ID NO: 60); SBP-tag, a peptide which binds to streptavidin (SEQ ID NO: 61); Softag 1, with SEQ ID NO: 62; Softag 3, with SEQ ID NO: 63; Spot-tag, a peptide recognized by a nanobody (SEQ ID NO: 64); Strep-tag, a peptide which binds to streptavidin or the modified streptavidin called streptactin (Strep-tag II has SEQ ID NO: 65); T7-tag, an epitope tag derived from the T7 major capsid protein of the T7 gene (SEQ ID NO: 66); TC tag, a tetracysteine tag that is recognized by FlAsH and ReAsH biarsenical compounds (SEQ ID NO: 67); Ty tag (SEQ ID NO: 68); V5 tag, (SEQ ID NO: 69); VSV-tag, (SEQ ID NO: 70); Xpress tag (SEQ ID NO: 71 DLYDDDDK); HiBiT-tag. Other peptide tags form covalent bonds with specific proteins, such as Isopeptag, a peptide which binds covalently to pilin-C protein (SEQ ID NO: 72 TDKDMTITFTNKKDAE); SpyTag, a peptide which binds covalently to SpyCatcher protein (SEQ ID NO: 73 AHIVMVDAYKPTK); SnoopTag, a peptide which binds covalently to SnoopCatcher protein (SEQ ID NO: 74 KLGDIEFIKVNK) or second generation, SnoopTagJr, developed to bind to either SnoopCatcher or DogTag (mediated by SnoopLigase) (SEQ ID NO: 75 KLGSIEFIKVNK); DogTag, a peptide which covalently binds to DogCatcher (SEQ ID NO: 76 DIPATYEFTDGKHYITNEPIPPK), and can also covalently bind to SnoopTagJr, mediated by SnoopLigase; SdyTag, a peptide which binds covalently to SdyCatcher protein (SEQ ID NO: 77 DPIVMIDNDKPIT); or chitin-binding domain (CBD)-tag. Other tags are proteins, such as BCCP (Biotin Carboxyl Carrier Protein), a protein domain biotinylated by BirA enabling recognition by streptavidin; FAST (Fluorescence-Activating and absorption-Shifting Tag), a mutated photoactive yellow protein (PYP) that reversibly binds cognate fluorogenic ligands; CL7-tag, an engineered variant of Colicin E7 that has a strong binding affinity and specificity for immobilized Immunity Protein 7 (Im7); Glutathione-S-transferase-tag, a protein which binds to immobilized glutathione; Green fluorescent protein-tag or GFP-tag, a protein which is spontaneously fluorescent and can be bound by nanobodies; HaloTag, a mutated bacterial haloalkane dehalogenase that covalently attaches to haloalkane substrates; SNAP-tag, a mutated eukaryotic DNA methyltransferase that covalently attaches to benzylguanine derivatives; CLIP-tag, a mutated eukaryotic DNA methyltransferase that covalently attaches to benzylcytosine derivatives, Maltose binding protein-tag or MBP-tag, a protein which binds to amylose agarose.

In a preferred embodiment of the first aspect of the present invention, the Fc region is selected from IgG, IgM, IgA, IgD and IgE. In a preferred embodiment, the Fc region is selected from IgG1, IgG2, IgG3, IgG4, IgM, IgA1, IgA2, IgD and IgE.

In a preferred embodiment of the first aspect of the present invention, the tag is selected from His-tag, Flag, HA-tag, Myc-tag, Streptag, GST-tag, GFP-tag, V5tag, MBP, SNAP-tag, chitin-binding domain-tag, Calmodulin-binding protein CBP-tag, SUMO-tag, PA-tag, Ty1 tag or any combination thereof. In a preferred embodiment, the tag is other than GFP. In a preferred embodiment, the tag is selected from His-tag, Flag, HA-tag, Myc-tag or a combination thereof. In a preferred embodiment, the tag is selected from His-tag, Myc-tag or a combination thereof. In a preferred embodiment, the tag is His-tag and it comprises at least 6 histidines, preferably it comprises 6 histidines (SEQ ID NO: 78).

In a preferred embodiment of the first aspect of the present invention, the Fc region origin is mammal. In a preferred embodiment, the origin of the Fc region is of any species relevant in research and veterinary, such as human, rat, mouse, pig, horse, non-human primate, cow, hamster, ferret, rabbit, dog or cat origin. Preferably, it is of human or rat or mouse origin.

Preferred embodiments of the first aspect are any one of the recombinant proteins encoded by SEQ ID NO: 29, SEQ ID NO: 31, SEQ ID NO: 33, SEQ ID NO: 35, SEQ ID NO: 37, SEQ ID NO: 39, SEQ ID NO: 41, SEQ ID NO: 43 and SEQ ID NO: 45.

In a preferred embodiment of the second aspect of the present invention, the nucleic acid is DNA, for example a vector, or RNA, for example mRNA codifying for the recombinant proteins of the first aspect.

In a preferred embodiment of the third aspect of the present invention, the cell can be either a prokaryotic or an eukaryotic cell. The skilled person is aware of the many cell alternatives which can produce the recombinant proteins of the first aspect of the present invention.

The fourth aspect of the present invention relates to a kit of parts comprising at least two recombinant proteins of the first aspect, or at least two nucleic acids of the second aspect, or at least two cells of the third aspect, wherein each recombinant protein comprises the variable part of an antibody against the same specific tag and the Fc region of each recombinant protein is from an antibody with a different isotype, for use as quantitation standard in an immunoassay for antibodies against antigens tagged with that specific tag. In a preferred embodiment, the kit of parts comprises at least two and up to 100 different recombinant proteins, nucleic acids or cells, preferably up to 80, up to 70 up to 60, up to 50, up to 40, up to 30, up to 20 or up to 10 different recombinant proteins, nucleic acids or cells. In a preferred embodiment, the kit of parts comprises from 2 to 10 or from 2 to 20 different recombinant proteins, nucleic acids or cells. The recombinant proteins in the kit of parts preferably have the Fc region of the same animal origin. A preferred kit of parts is for example one with 9 different recombinant proteins of the first aspect or nucleic acids of the second aspect or cells of the third aspect, where the Fc region is of human origin and the isotypes are IgG1, IgG2, IgG3, IgG4, IgM, IgA1, IgA2, IgD and IgE. Another preferred kit would be any kit derived from this one, with at least two and up to eight different recombinant proteins. Another example of preferred kit of parts is one where the Fc region is of mouse origin and the isotypes are IgG1, IgG2a, IgG2b, IgG2c and IgG3, or any kit derived from this one with at least two and up to four different recombinant proteins. Another example of preferred kit of parts is one where the Fc region is of rat origin and the isotypes are IgG1, IgG2a, IgG2b and IgG2c, or any kit derived from this one with at least two and up to three different recombinant proteins. Other preferred kit of parts comprises human isotypes IgM, IgG and IgA. Other comprise human isotypes IgG1, IgG2, IgG3 and IgG4. Other comprise human isotypes IgA1 and IgA2. Other comprise isotypes human IgG4 and IgE. Other comprise isotypes human IgM and IgD. Also, preferred kits are custom designed kits where the tag, the origin species and the antibody isotypes are chosen.

In a preferred embodiment of the fourth aspect of the present invention, the kit of parts of the fourth aspect further comprising at least one immunoassay reagent. Immunoassay reagents can be chosen between buffers, such as dilution buffers, blocking buffers, washing buffers or any sort of buffer, secondary antibodies according to the selected species of the Fc region, isolated or on precoated plastic plates, as well as reagents for developing a signal and/or stopping the reaction to develop said signal.

In a preferred embodiment of all the previous aspects, which are for use as quantitation standard in an immunoassay for antibodies against antigens tagged with that specific tag, the immunoassay is an ELISA. ELISA can be direct, indirect, sandwich or competitive.

In another preferred embodiment of all the previous aspects, which are for use as quantitation standard in an immunoassay for antibodies against antigens tagged with that specific tag, the immunoassay is Western Blot.

In another preferred embodiment of all the previous aspects, which are for use as quantitation standard in an immunoassay for antibodies against antigens tagged with that specific tag, the immunoassay is a bead-based assay.

In another preferred embodiment of all the previous aspects, which are for use as quantitation standard in an immunoassay for antibodies against antigens tagged with that specific tag, the immunoassay is an antibody dependent cellular cytotoxicity assay.

In another preferred embodiment of all the previous aspects, which are for use as quantitation standard in an immunoassay for antibodies against antigens tagged with that specific tag, the immunoassay is an antibody-dependent cellular phagocytosis assay.

Other types of immunoassays for which the present invention is useful are FRET-Based Immunoassays (Fluorescence resonance energy transfer (FRET) immunoassays), Lumit Immunoassays based on NanoBiT technology (a protein complementation system that uses two subunits of an engineered luciferase enzyme: small bit (SmBiT) and large bit (LgBiT)).

All the above embodiments of the first aspect also apply to the second, third and fourth aspects. All the embodiments of the second aspect apply also to the first, third and fourth aspects. All the embodiments of the third aspect apply also to the first, second and fourth aspects. All the embodiments of the fourth aspect apply also to the first, second and third aspects. The present disclosure includes all the combinations of all the aspects disclosed above. The term "comprising" or "comprise" or "comprises" includes the term "consisting of" or "consist of" or "consists of".

### DESCRIPTION OF THE FIGURES

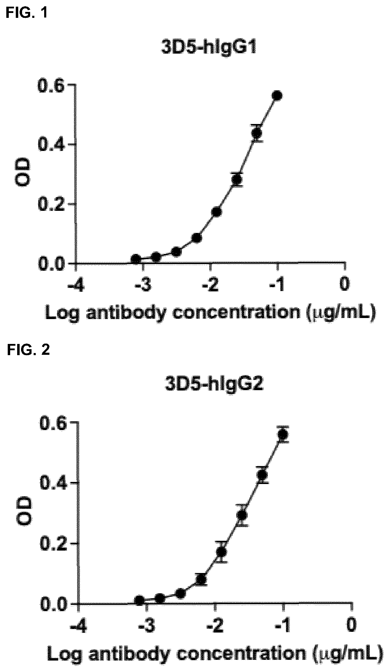

**Figure 1****. Binding of 3D5-hIgG1 to a 6xHis-tagged antigen determined by ELISA.** Eight 1/3 serial dilution of the antibody starting at 0.1 µg/mL in blocking buffer (PBS+1 %BSA) were assayed. OD= optical density. Bound antibodies were detected using a mouse anti-human kappa monoclonal antibody followed by a secondary HRP-conjugated anti-mouse IgG antibody. OPD was used as substrate.
**Figure 2****. Binding of 3D5-higG2 to a 6xHis-tagged antigen determined by ELISA.** Eight 1/3 serial dilution of the antibody starting at 0.1 µg/mL in blocking buffer (PBS+1 %BSA) were assayed. OD= optical density. Bound antibodies were detected using a mouse anti-human kappa monoclonal antibody followed by a secondary HRP-conjugated anti-mouse IgG antibody. OPD was used as substrate.

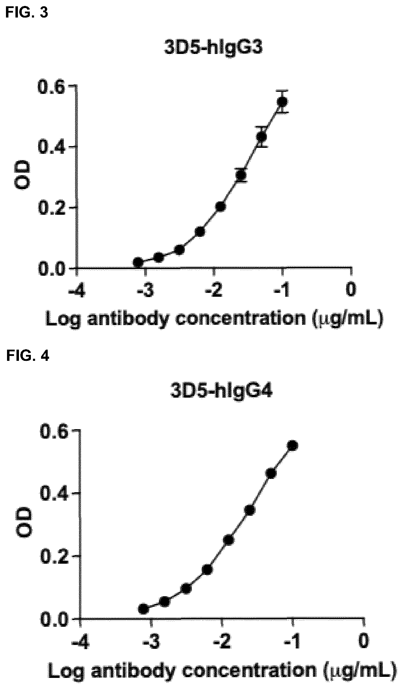

**Figure 3****. Binding of 3D5-higG3 to a 6xHis-tagged antigen determined by ELISA.** Eight 1/3 serial dilution of the antibody starting at 0.1 µg/mL in blocking buffer (PBS+1 %BSA) were assayed. OD= optical density. Bound antibodies were detected using a mouse anti-human kappa monoclonal antibody followed by a secondary HRP-conjugated anti-mouse IgG antibody. OPD was used as substrate.
**Figure 4****. Binding of 3D5-higG4 to a 6xHis-tagged antigen determined by ELISA.** Eight 1/3 serial dilution of the antibody starting at 0.1 µg/mL in blocking buffer (PBS+1 %BSA) were assayed. OD= optical density. Bound antibodies were detected using a mouse anti-human kappa monoclonal antibody followed by a secondary HRP-conjugated anti-mouse IgG antibody. OPD was used as substrate.

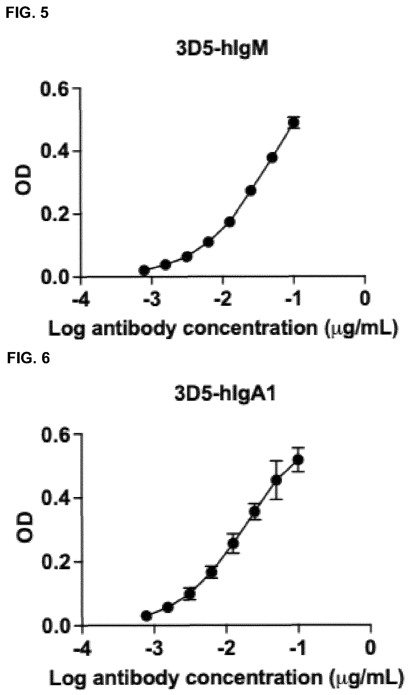

**Figure 5****. Binding of 3D5-hIgM to a 6xHis-tagged antigen determined by ELISA.** Eight 1/3 serial dilution of the antibody starting at 0.1 µg/mL in blocking buffer (PBS+1 %BSA) were assayed. OD= optical density. Bound antibodies were detected using a mouse anti-human kappa monoclonal antibody followed by a secondary HRP-conjugated anti-mouse IgG antibody. OPD was used as substrate.
**Figure 6****. Binding of 3D5-hIgA1 to a 6xHis-tagged antigen determined by ELISA.** Eight 1/3 serial dilution of the antibody starting at 0.1 µg/mL in blocking buffer (PBS+1 %BSA) were assayed. OD= optical density. Bound antibodies were detected using a mouse anti-human kappa monoclonal antibody followed by a secondary HRP-conjugated anti-mouse IgG antibody. OPD was used as substrate.

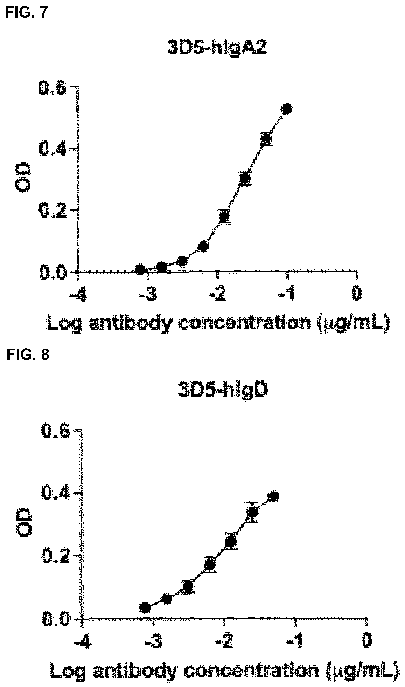

**Figure 7****. Binding of 3D5-higA2 to a 6xHis-tagged antigen determined by ELISA.** Eight 1/3 serial dilution of the antibody starting at 0.1 µg/mL in blocking buffer (PBS+1 %BSA) were assayed. OD= optical density. Bound antibodies were detected using a mouse anti-human kappa monoclonal antibody followed by a secondary HRP-conjugated anti-mouse IgG antibody. OPD was used as substrate.
**Figure 8****. Binding of 3D5-hIgD to a 6xHis-tagged antigen determined by ELISA.** Eight 1/3 serial dilution of the antibody starting at 0.05µg/mL in blocking buffer (PBS+1%BSA) were assayed. OD= optical density. Bound antibodies were detected using a mouse anti-human kappa monoclonal antibody followed by a secondary HRP-conjugated anti-mouse IgG antibody. OPD was used as substrate.

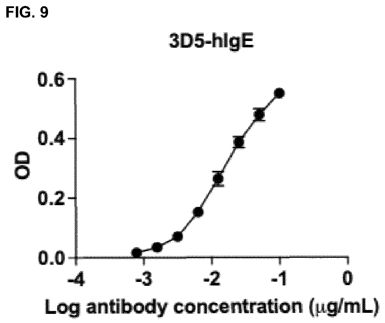

**Figure 9****. Binding of 3D5-hIgE to a 6xHis-tagged antigen determined by ELISA.** Eight 1/3 serial dilution of the antibody starting at 0.1 µg/mL in blocking buffer (PBS+1 %BSA) were assayed. OD= optical density. Bound antibodies were detected using a mouse anti-human kappa monoclonal antibody followed by a secondary HRP-conjugated anti-mouse IgG antibody. OPD was used as substrate.

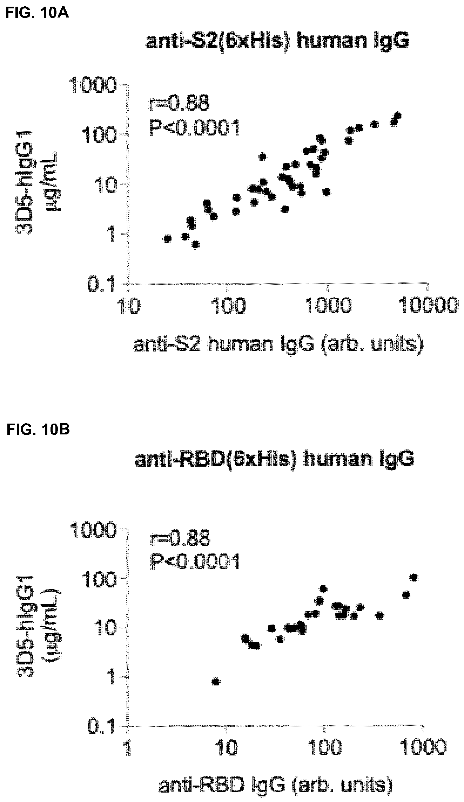

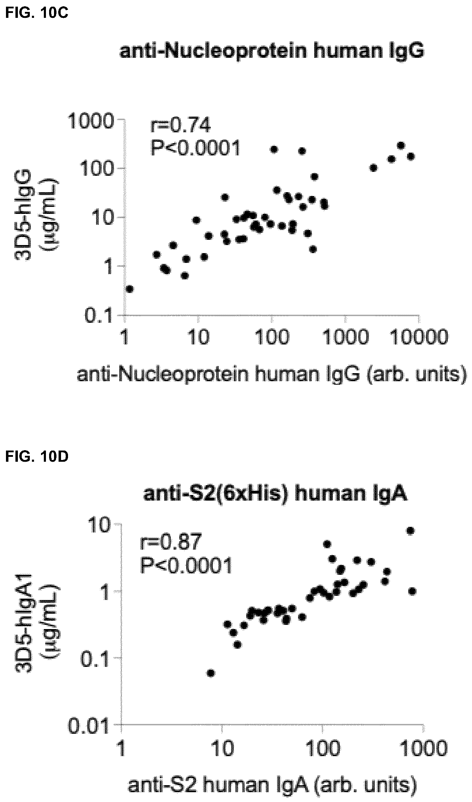

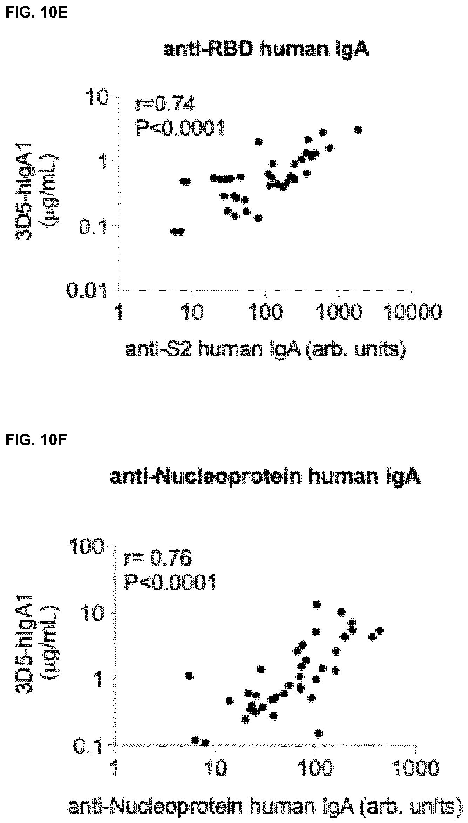

**Figure 10****. Correlation analysis of anti-SARS-CoV-2 S2, RBD and Nucleoprotein IgG and IgA humoral responses using 3D5 Abs and human plasma as standard.** IgG (A, B and C) and IgA (D, E and F) humoral responses to SARS-CoV-2 S2 (A and D), RBD (B and E) and Nucleoprotein (C and F) were determined by ELISA using selected plasma samples from SARS-CoV-2 convalescent individuals as standard. Results are shown as arbitrary units (arb. units)/mL. In parallel, the same samples were analyzed using 3D5-hIgG1 (A, B and C), and 3D5-hlgA1 (D, E and F). Results are showed as µg/mL. Spearman correlation (two-tailed) analysis was performed. Spearman r and P value are shown.

### EXAMPLES

The following examples illustrate the present invention and are not to be understood as limiting the invention.

### Example 1: Generation of chimeric anti-His-tag (6 histidines) 3D5-human Fc (IgG₁₋₄, IgA₁₋₂, IgM, IgD and IgE) bearing Kappa or Lambda light chains

The amino acid sequence of both heavy chain and light chain variable regions were obtained from the Protein Data Bank under the accession number 1KTR. Gene fragments were codon optimized and ordered from Integrated DNA Technologies (IDT) or GeneArt (ThermoFisher Scientific). The VH gene (SEQ ID NO: 1) was cloned into human Fc expressing vectors (all isotypes and subclasses: IgA₁ (SEQ ID NO: 3), IgA₂ (SEQ ID NO: 5), IgD (SEQ ID NO: 7), IgE (SEQ ID NO: 9), IgG₁ (SEQ ID NO: 11), IgG₂ (SEQ ID NO: 13), IgG₃ (SEQ ID NO: 15), IgG₄ (SEQ ID NO: 17), IgM (SEQ ID NO: 19) in a pcDNA3.4 backbone using Kpnl and Nhel. The VL gene (SEQ ID NO: 21) was cloned into a human Kappa (SEQ ID NO: 23) or Lambda (SEQ ID NO: 25) expressing vectors in a pcDNA3.4 background (ThermoFisher scientific). The sequence of the IL-2 signal peptide (SEQ ID NO: 27) was added in the N-terminal region to favor soluble expression. After cloning, sequences were confirmed by sequencing.

The sequences of the obtained antibodies are SEQ ID NO: 29, SEQ ID NO: 31, SEQ ID NO: 33, SEQ ID NO: 35, SEQ ID NO: 37, SEQ ID NO: 39, SEQ ID NO: 41, SEQ ID NO: 43 and SEQ ID NO: 45. The sequences of the 3D5 hulg Kappa and 3D5 hulg Lambda are SEQ ID NO: 47 and SEQ ID NO: 49, respectively.

Antibodies were produced by transient transfection in Expi293 (ThermoFisher scientific) following the manufacture's protocol. Recombinant antibodies were purified using standard affinity chromatography techniques such as protein-A/G chromatography (for human IgGs) (Cytiva), CaptureSelect IgA-XL Affinity Matrix (for human IgAs) (ThermoFisher scientific), CaptureSelect KappaXP (ThermoFisher scientific) (for all isotypes bearing a kappa light chain) or CaptureSelect^{™} LambdaXP Affinity Matrix (ThermoFisher scientific) (for all isotypes bearing a human Lambda light chain).

### Example 2: Quantification of 3D5-recombinant human antibodies by Enzyme-Linked Immuno Sorbent Assay (ELISA) using isotype specific secondary antibodies.

Plates were coated with mouse anti-kappa or anti-lambda monoclonal antibodies (cat: 316502 and 316602, respectively, Biolegend) at 2 µg/ml and incubated at 4°C overnight. The next day, plates were washed and 1% bovine serum albumin (BSA) in PBS (blocking buffer) was added and incubated for two hours at room temperature. Serially diluted supernatants from Expi293 cells expressing the different antibodies were added and incubated for one hour at room temperature. HRP-conjugated secondary antibodies specific for each isotype were used for the detection of: human IgG (cat: 109-036-098, Jackson ImmunoResearch), human IgA (cat: 109-035-011, Jackson ImmunoResearch) and human IgM (cat: 109-036-129, Jackson ImmunoResearch). For the detection of IgD and IgE, the following biotin-conjugated secondary antibodies were used: human IgD (cat. 2030-08, Southern Biotech), human IgE (cat: 9160-08, Southern Biotech) plus HRP streptavidin (cat: 890803, R&D Systems). All wells were developed with o-phenylenediamine dihydrochloride (OPD, cat: P8412-50TAB, Sigma Aldrich). The reaction was stopped with 2N H₂SO₄ and all plates were read at 492nm with noise correction at 620nm.

### Example 3: Performance evaluation of the 3D5-recombinant human antibodies to recognize proteins tagged with a His-tag

Plates were coated with SARS-CoV-2 receptor binding domain (RBD)-HIS (cat: 40592-V08H, SinoBiological) at 2 µg/ml and incubated at 4°C overnight. The next day, plates were washed and blocked using 1% BSA in 1x PBS (blocking buffer) for two hours at room temperature. Serially diluted supernatants in blocking buffer were added and incubated overnight at 4°C. Purified 3D5-human IgG1 antibody was used as standard. After washing, purified mouse anti-human kappa (cat: 316502, Biolegend) at 1 µg/ml was added to all wells and incubated for one hour at room temperature. Next, we added HRP-goat anti-mouse IgG (cat:115-036-071, Jackson ImmunoResearch) and incubated for 30 minutes. Lastly, plates were developed with o-phenylenediamine dihydrochloride (OPD, cat: P8412-50TAB, Sigma Aldrich). The reaction was stopped with 2N H₂SO₄ and all plates were read at 492nm with noise correction at 620nm.

Figures 1 to 9 show the signal (optical density, OD) versus standard concentration (in Log scale) of all 3D5-human chimeric molecules using an 6xHis-tagged antigen. A comparable performance among all standard is observed.

### Example 4: Antigen-specific human IgG, IgA, and IgM ELISA

Half plates were coated at 4°C overnight with the following SARS-CoV-2 recombinant antigens tagged with 6xhis: RBD (cat: 40592-V08H, SinoBiological), Spike subunit S2 (cat: 40590-V08B, SinoBiological), and Nucleocapsid protein (cat: 40588-V08B, SinoBiological) at 1 µg/ml in PBS. The next day, plates were washed and blocked with 1% BSA in 1x PBS (blocking buffer) for two hours at room temperature. Serially diluted purified 3D5 IgG and IgA starting at 1 µg/ml were used as standards. Diluted plasmas, standards, and controls were then added to antigen-free and antigen-coated wells and incubated overnight at 4°C. After washing, HRP goat anti-human IgG, or IgA antibodies (cat: 109-036-098, and 109-035-011, respectively, Jackson ImmunoResearch) were added. Plates were incubated for 30 minutes at room temperature and washed again. Lastly, plates were developed with OPD (cat: P8412-50TAB, Sigma Aldrich). The reaction was stopped with 2N H₂SO₄ and all plates were read at 429nm with noise correction at 620nm.

Figures 10A to 10F display a correlation analysis between antigen-specific antibody levels quantified using the 3D5-derived human IgG and IgA standards (expressed in microgram/mL), and those obtained using a positive human plasma sample used as reference (showed as arbitrary units/mL). IgG and IgA human antibodies targeting SARS-CoV-2 Spike subunit 2 (S2), SARS-CoV-2 Receptor Binding Domain (RBD) and SARS-CoV-2 Nucleoprotein (NP) were quantified as described above. Results showed a strong positive correlation between both assays.

## Claims

1. A recombinant protein comprising the variable part of an antibody against a specific tag and the Fc region of an antibody, for use as quantitation standard in an immunoassay for antibodies against antigens tagged with that specific tag.

2. The recombinant protein of claim 1, wherein the Fc region is selected from IgG, IgM, IgA, IgD and IgE.

3. The recombinant protein of any one of claims 1 or 2, wherein the tag is selected from His-tag, Flag, HA, Myc, Streptag, GST, GFP, V5tag, MBP, SNAP-tag, chitin-binding domain (CBD)-tag, Calmodulin-binding protein (CBP)-tag, SUMO-tag, PA-tag, Ty1 tag or any combination thereof.

4. The recombinant protein of any one of claims 1 to 3, wherein the tag is selected from His-tag, Flag, HA, Myc or any combination thereof.

5. The recombinant protein of any one of claims 1 to 3, wherein the Fc region origin is mammal.

6. A nucleic acid encoding for the recombinant protein of any one of claims 1 to 5, for use as quantitation standard in an immunoassay for antibodies against antigens tagged with that specific tag.

7. A cell comprising the nucleic acid of claim 6, for use as quantitation standard in an immunoassay for antibodies against antigens tagged with that specific tag.

8. A kit of parts comprising at least two recombinant proteins according to any one of claims 1 to 5, or at least two nucleic acids according to claim 6, or at least two cells according to claim 7, wherein each recombinant protein comprises the variable part of an antibody against the same specific tag and the Fc region of each recombinant protein is from an antibody with a different isotype.

9. The kit of parts of claim 8, further comprising at least one immunoassay reagent.

10. The kit of parts of any one of claims 8 or 9, for use as quantitation standard in an immunoassay for antibodies against antigens tagged with that specific tag.

11. The recombinant protein of any one of claims 1 to 5, the nucleic acid of claim 6, the cell of claim 7 or the kit of parts of any one of claims 8 or 9, for use as quantitation standard in an immunoassay for antibodies against antigens tagged with that specific tag, wherein the immunoassay is an ELISA.

12. The recombinant protein of any one of claims 1 to 5, the nucleic acid of claim 6, the cell of claim 7 or the kit of parts of any one of claims 8 or 9, for use as quantitation standard in an immunoassay for antibodies against antigens tagged with that specific tag, wherein the immunoassay is Western Blot.

13. The recombinant protein of any one of claims 1 to 5, the nucleic acid of claim 6, the cell of claim 7 or the kit of parts of any one of claims 8 or 9, for use as quantitation standard in an immunoassay for antibodies against antigens tagged with that specific tag, wherein the immunoassay is a bead-based assay.

14. The recombinant protein of any one of claims 1 to 5, the nucleic acid of claim 6, the cell of claim 7 or the kit of parts of any one of claims 8 or 9, for use as quantitation standard in an immunoassay for antibodies against antigens tagged with that specific tag, wherein the immunoassay is an antibody dependent cellular cytotoxicity assay.

15. The recombinant protein of any one of claims 1 to 5, the nucleic acid of claim 6, the cell of claim 7 or the kit of parts of any one of claims 8 or 9, for use as quantitation standard in an immunoassay for antibodies against antigens tagged with that specific tag, wherein the immunoassay is an antibody-dependent cellular phagocytosis assay.
